(19) Europäisches Patentamt
European Patent Office
Office européen des brevets

(11) Veröffentlichungsnummer: 0 287 908
A2

(12) EUROPÄISCHE PATENTANMELDUNG

(21) Anmeldenummer: 88105651.9

(22) Anmeldetag: 08.04.88

(51) Int. Cl.4: C07D 401/12 , A61K 31/445

(30) Priorität: 18.04.87 DE 3713246

(43) Veröffentlichungstag der Anmeldung: 26.10.88 Patentblatt 88/43

(84) Benannte Vertragsstaaten: AT BE CH DE ES FR GB IT LI LU NL SE

(71) Anmelder: ASTA Pharma AG
Weismüllerstrasse 45
D-6000 Frankfurt am Main 1(DE)

(72) Erfinder: Engel, Jürgen, Dr.
Erlenweg 3
D-8755 Alzenau(DE)
Erfinder: Kleemann, Axel, Prof. Dr.
Bornweg 36
D-6052 Mühlheim(DE)
Erfinder: Nickel, Bernd, Dr.
Alleestrasse 35
D-6109 Mühltal(DE)
Erfinder: Szelenyi, Istvan, Dr.
Händelstrasse 32
D-8501 Schwaig(DE)

(54) **Neue stickstoffhaltige, cycloaliphatische Verbindungen mit einem Aminosäurerest sowie einem Pyridinrest.**

(57) Verbindungen der Formel

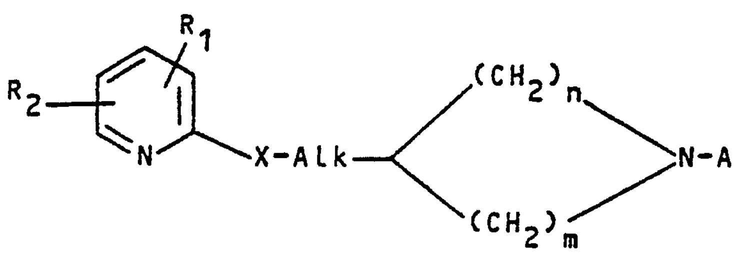

I

worin die Reste $R_1$ und $R_2$ gleich oder verschieden sind und Wasserstoff, Halogenatome, eine Trifluormethylgruppe, eine Cyanogruppe, eine Nitrogruppe, eine Aminogruppe, eine Mono-$C_1$-$C_6$-alkylaminogruppe, eine Di-$C_1$-$C_6$-alkylaminogruppe, eine Aminogruppe, die durch einen Phenyl-$C_1$-$C_4$-alkylrest oder einen Halogenphenyl-$C_1$-$C_4$-alkylrest substituiert ist, eine $C_2$-$C_6$-Alkanoylaminogruppe, eine $C_1$-$C_6$-Alkoxy carbonylaminogruppe, eine gegebenenfalls durch einen Phenylrest substituierte $C_1$-$C_6$-Alkylgruppe, eine Hydroxygruppe, eine $C_1$-$C_6$-Alkoxygruppe, eine $C_2$-$C_6$-Alkanoyloxygruppe, eine Phenoxygruppe oder eine gegebenenfalls durch eine oder zwei $C_1$-$C_6$-Alkylgruppen substituierte Carbamoylgruppe bedeuten und der Rest A eine Acylgruppe ist, die sich von einer Aminosäure ableitet, sowie Verfahren zu deren Herstellung.

EP 0 287 908 A2

# Neue stickstoffhaltige, cycloaliphatische Verbindungen mit einem Aminosäurerest sowie einem Pyridinrest

In der Europa-Anmeldung 0 149 088 werden analgetisch wirksame Verbindungen der Formel

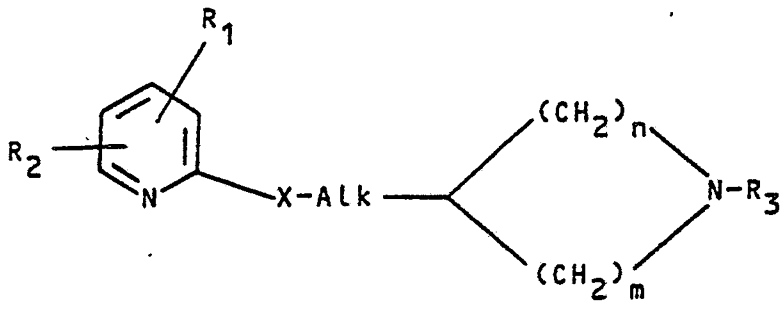

beschrieben, worin die Reste $R_1$ und $R_2$ gleich oder verschieden sind und Wasserstoff, Halogenatome, eine Trifluormethylgruppe, eine Cyanogruppe, eine Nitrogruppe, eine Aminogruppe, eine Mono-$C_1$-$C_6$-alkylaminogruppe, eine Di-$C_1$-$C_6$-alkylaminogruppe, eine Aminogruppe, die durch einen Phenylrest, einen Mono- oder Dihalogenphenylrest oder einen Phenyl-$C_1$-$C_4$-alkylrest substituiert ist, eine $C_1$-$C_6$-Alkanoylamino gruppe, eine $C_1$-$C_6$-Alkoxycarbonylaminogruppe, eine gegebenenfalls durch einen Phenylrest substituierte $C_1$-$C_6$-Alkylgruppe, eine Phenylgruppe, eine Hydroxygruppe, eine $C_1$-$C_6$-Alkoxygruppe, eine Phenoxygruppe, eine Carboxygruppe, eine Carb-$C_1$-$C_6$-alkoxygruppe oder eine gegebenenfalls durch eine oder zwei $C_1$-$C_6$-Alkylgruppen substituierte Carbamoylgruppe bedeuten, der Rest $R_3$ Wasserstoff, eine $C_1$-$C_6$-Alkylgruppe, eine $C_3$-$C_6$-Alkenylgruppe, eine $C_3$-$C_6$-Alkinylgruppe, eine $C_3$-$C_7$-Cycloalkylgruppe, eine $C_5$-$C_7$-Cycloalkenylgruppe, eine Phenyl-$C_1$-$C_4$-alkylgruppe, eine Carb-$C_1$-$C_6$-alkoxygruppe, eine gegebenenfalls durch einen $C_3$-$C_6$-Cycloalkylrest substituierte $C_2$-$C_6$-Alkanoylgruppe oder eine $C_1$-$C_4$-Alkylgruppe ist, die am selben C-Atom zwei $C_1$-$C_6$-Alkoxygruppen oder eine $C_2$-$C_4$-Alkylendioxygruppe enthält oder worin $R_3$ eine $C_1$-$C_6$-Alkylgruppe bedeutet, die ein-oder zweifach durch $C_3$-$C_7$-Cycloalkylgruppen, Hydroxygruppen, $C_1$-$C_6$-Alkoxygruppen, Halogenatome, Sulfogruppen ($-SO_3H$), Aminogruppen, $C_1$-$C_6$-Alkylaminogruppen, Di-$C_1$-$C_6$-alkylaminogruppen, $C_1$-$C_6$-Alkylcarbonylgruppen, $C_3$-$C_7$-Cycloalkylcarbonylgruppen, Carb-$C_1$-$C_6$-alkoxygruppen oder Benzoylgruppen substituiert ist, X Sauerstoff, Schwefel, SO oder $SO_2$ bedeutet, Alk Alkylen mit 0-4 C-Atomen ist und n und m gleich oder verschieden sind und die Zahlen 1-3 annehmen können, wobei n auch O sein kann, wenn Alk Alkylen mit mindestens einem Kohlenstoffatom ist und m für diesen Fall die Zahlen 2-6 annimmt und wobei die Gruppierung

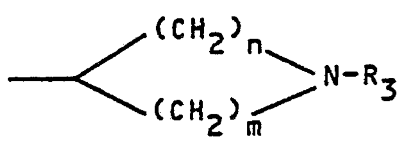

auch den Chinuclidylrest oder den Tropanylrest darstellen kann.

Die erfindungsgemäßen Verbindungen sind pharmakologisch wirksam. Insbesondere besitzen die erfindungsgemäßen Verbindungen eine ausgeprägte und starke analgetische Wirkung. Im Vergleich zu den vorbekannten Verbindungen ist diese analgetische Wirkung insbesondere lang anhaltend und setzt verzögernd ein.

Der Erfindung liegt also die Aufgabe zugrunde, Verbindungen mit günstigen pharmakologischen Eigenschaften zur Verfügung zu stellen, die beispielsweise als analgetisch wirkende Arzneimittel (insbesondere als Retard-Mittel) verwertbar sind.

Die folgenden Erläuterungen stellen erfindungswesentliche Angaben dar:

Die in der Formel I vorkommenden Alkylgruppen, Alkoxygruppen, Alkylaminogruppen, Alkanoylaminogruppen, Alkanoyloxygruppen, Alkanoylmercaptogruppen beziehungsweise ganz allgemein Alkanoylgruppen können gerade oder verzweigt sein. Dasselbe gilt auch für Alkyl-und Alkyloxygruppen (= Alkoxygruppen), falls diese Bestandteil anderer zusammengesetzter Gruppen sind (zum Beispiel in Form einer Monoalkyl- oder Dialkylaminogruppe, Alkanoylaminogruppe, Alkoxycarbonylaminogruppe, Carbalkoxygruppe, Alkylcarbonylgruppe, Aminoalkyloxygruppe und analoge Gruppen. Desgleichen kann bei der Bedeutung Phenyl-$C_1$-$C_4$-alkylrest(gruppe) der Alkylteil, falls dieser aus 2-4 C-Atomen besteht, ebenfalls gerade oder verzweigt sein. Bei den Halogenatomen handelt es sich um Chlor, Brom oder Fluor, insbesondere Chlor und Fluor. Die Alkyl-und Alkoxygruppen als solche oder als Bestandteil von anderen zusammengesetzten Gruppen bestehen insbesondere aus 1-4 C-Atomen, vorzugsweise 1 oder 2 C-Atomen. Alkanoylgruppen, wie zum Beispiel Alkanoylaminogruppen, Alkanoyloxygruppen oder Alkanoylmercaptorgruppen, bestehen insbesondere aus 2-4, vorzugsweise 2-3 C-Atomen. Der Alkylteil des Phenyl-$C_1$-$C_4$-alkylrestes(gruppe) besteht insbesondere aus 1-3, vorzugsweise 1 oder 2 C-Atomen. Die Gruppe

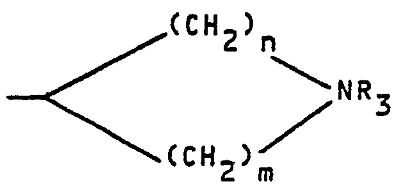

bildet insbesondere einen 5-, 6-oder 7-Ring.

Beispiele hierfür sind: Piperidinring (Piperidyl-(4)-, Piperidyl-(3)-oder Piperidyl-(2)-ring), Homopiperidinring (zum Beispiel Homopiperidyl-(4)-ring), Pyrrolidinring (Pyrrolidyl-(2)-oder Pyrrolidyl-(3)-ring).

X bedeutet vorzugsweise Schwefel.

Besonders wichtig sind solche Verbindungen der Formel I, worin X Schwefel, einer der Reste $R_1$ beziehungsweise $R_2$ Wasserstoff und der gesättigte stickstoffhaltige Ring ein Piperidylrest ist, der direkt mit dem Schwefelatom verbunden ist (Alk = 0 C-Atome, das heißt Alk entfällt) und $R_3$ Wasserstoff, Phenyl, Phenyl-$C_1$-$C_4$-alkyl, eine gerade oder verzweigte $C_1$-$C_6$-Alkylgruppe darstellt, die auch eine Mercaptogruppe, eine $C_1$-$C_4$-Alkylmercaptogruppe, eine Aminogruppe, eine Carboxygruppe, eine Guanidinogruppe oder die Gruppe -$CONH_2$ enthalten kann. Hierbei enthält der Pyridinring vorzugsweise einen Substituenten entsprechend den angegebenen Bedeutungen für $R_1/R_2$, vorzugsweise ist dieser Substituent ein Halogenatom (beispielsweise Chlor), eine Trifluormethylgruppe oder eine $C_1$-$C_6$-Alkylgruppe (zum Beispiel $CH_3$), welche sich insbesondere in 6-Stellung des Pyridinringes befindet. Falls Alk vorhanden ist, besteht diese Gruppe insbesondere aus 1 oder 2 C-Atomen.

$R_3$ ist beispielsweise eine $C_1$-$C_6$-Alkylgruppe, die in 1-, 2-, 3-, 4-, 5-oder 6-Stellung (Zählung beginnt stets an der Verknüpfungsstelle des Alkylrestes mit dem Restmolekül) eine Aminogruppe (insbesondere in 3-oder 4-Stellung), eine Mercapto-oder Hydroxygruppe (insbesondere in 1-oder 2-Stellung), eine Amino-$C_2$-$C_4$-alkylthiogruppe, eine Amino-$C_2$-$C_4$-alkoxygruppe, eine Carboxygruppe, eine $C_1$-$C_6$-Alkoxycarbonylgruppe, eine Ureidogruppe oder einen Guanidinorest enthält. Beispielsweise liegen der Gruppe -CO-CH-($NR_4R_5$)-$R_3$ beziehungsweise -CO-CH($NR_4R_6$)-$R_3$ die folgenden Aminosäuren zugrunde: Asparaginsäure (DL-Form), Asparagin, $\alpha$-Amino-buttersäure, Leucin, Isoleucin, Asparaginsäureethylester (L-Form), Citrullin ($H_2N$-CO-NH-$(CH_2)_3$-CH($NH_2$)-$CO_2H$, L-Form), Ornithin (L-Form), Arginin, 4-Thialysin ($H_2N$-$CH_2$-$CH_2$-S-$CH_2$-CH($NH_2$)-COOH), 2,6-Diamino-önanthsäure ($\epsilon$-Methyllysin), 4-Oxalysin ($H_2N$-$CH_2CH_2$-O-$CH_2$-CH($NH_2$)-COOH), Glycin, N-Methyl-glycin, N,N-Dimethylglycin, Prolin, Hydroxyprolin, Alanin, $\beta$-Alanin, 3,4-Dihydroxyphenylalanin, Phenylalanin, Tyrosin, Tryptophan, Cystein, Homocystein (DL-Form), Methionin, Penicillamin, Lysin (insbesondere L-Lysin), Valin, Valinmethylester(L-Form)

Threonin, Histidin, Serin, Homoserin, Glutaminsäure, Glutamin, $\alpha,\beta$-Diaminopropionsäure, Sarkosin, Ethionin, $\alpha,\gamma$-Diamino-buttersäure (L-Form), $\alpha$-Aminoadipinsäure (L-Form).

Besonders günstige Wirkungen zeigen beispielsweise Verbindungen der folgenden Formel

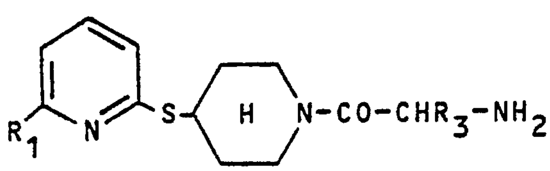

worin $R_1$ Chlor, Brom, Fluor oder $CF_3$ ist und $R_3$ Wasserstoff, Phenyl, Benzyl oder $C_1$-$C_6$-Alkyl ist, wobei der $C_1$-$C_6$-Alkylrest auch eine Mercaptogruppe, eine $C_1$-$C_4$-Alkylmercaptogruppe, eine Hydroxygruppe oder eine $C_1$-$C_4$-Alkoxygruppe enthalten kann.

Gemäß dem Herstellungsverfahren können in den Verfahrensprodukten vorhandene Hydroxy-,

Mercapto-und/oder primäre beziehungsweise sekundäre Aminogruppen alkyliert oder acyliert werden. Die Alkylierung erfolgt beispielsweise durch Umsetzung mit Verbindungen der Formel MHal, $ArSO_2OM$ und $SO_2(OM)_2$, wobei Hal ein Halogenatom (insbensondere Chlor, Brom oder Jod) und Ar ein aromatischer Rest (zum Beispiel ein gegebenenfalls durch einen oder mehrere niedere Alkylreste substituierter Phenyl-oder Naphthylrest und M ein $C_1$-$C_6$-Alkylrest, eine Phenyl-$C_1$-$C_4$-alkylrest, ein Halogenphenyl-$C_1$-$C_4$-alkylrest oder ein Amino-$C_1$-$C_6$-alkylrest mit geschützter Aminogruppe ist. Beispiele sind p-Toluolsulfonsäure-$C_1$-$C_6$-alkylester, $C_1$-$C_6$-Dialkylsulfate, $C_1$-$C_6$-Alkylhalogenide und entsprechende. Bei den zuvor genannten Verbindungen kann die Alkylgruppe gerade oder verzweigt sein. Die Alkylierungs-und Acylierungsreaktion wird gegebenenfalls unter Zusatz von üblichen säurebindenden Mitteln, wie Alkalihydroxiden, Alkalicarbonaten, Alkalihydrogencarbonaten, Erdalkalicarbonaten, Alkaliacetaten, tertiären Aminen (zum Beispiel Trialkylaminen wie Triethylamin), Pyridin oder auch Alkalihydriden bei Temperaturen zwischen 0 und 200° C, vorzugsweise 40 und 140° C in inerten Lösungsmitteln oder Suspensionsmitteln durchgeführt. Als Lösungs- oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid, aliphatische Ether wie zum Beispiel Butylether; cyclische Ether wie zum Beispiel Tetrahydrofuran, Dioxan; Sulfoxide wie zum Beispiel Dimethylsulfoxid; tertiäre Säureamide wie zum Beispiel Dimethylformamid, N-Methyl-pyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe wie Cyclohexan und ähnliche. Auch wässrige Mischungen der genannten Lösungsmittel können verwendet werden. Häufig arbeitet man bei der Rückflußtemperatur der verwendeten Lösungsbeziehungsweise Dispergiermittel. Häufig werden die Alkylierungsreaktionskomponenten im Überschuß eingesetzt. Die Alkylierung kann auch in Gegenwart von Tetraalkylammoniumsalzen (insbesondere der Halogenide) in Kombination mit Alkalihydroxiden bei Temperaturen zwischen 0 - 100° C, vorzugsweise 20 - 80° C in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-MethylPyrrolidon, Hexamethylphosäuretriamid), Dimethylsulfoxid, Acetonitril, Dimethoxyethan, Aceton, Tetrahydrofuran.

Bei der Acylierung wird zum Beispiel eine $C_2$-$C_6$-Alkanoylgruppe, eine $C_1$-$C_6$-Alkylcarbonylgruppe, eine gegebenenfalls durch eine oder zwei $C_1$-$C_6$-Alkylreste substituierte Carbamoylgruppe oder die Gruppe -CO-CH($NR_4R_6$)-$R_3$ eingeführt. Man verfährt hierbei in an sich bekannter Weise vorzugsweise unter Verwendung der estprechenden Halogenide (zum Beispiel Carb-$C_1$-$C_6$-alkoxyhalogeniden, $C_2$-$C_6$-Alkanoylhalogeniden), der entsprechenden Anhydride oder auch unter Verwendung der entsprechenden Säuren in Gegenwart von bekannten Kondensationsmitteln (siehe zum Beispiel Verfahren a)). Die Reaktionstemperaturen liegen beispielsweise zwischen 30 und 120° C.

Gegebenenfalls kann man bei der Alkylierung und der Acylierung auch so vorgehen, daß man zuerst von der zu alkylierenden beziehungsweise acylierenden Verbindung eine Alkaliverbindung (Natrium-, Kalium-oder auch Lithiumsalz zum Beispiel) herstellt, indem man sie in einem inerten Lösungsmitel wie Dioxan, Dimethylformamid, Benzol oder Toluol mit einem Alkalimetall, Alkalihydrid oder Alkaliamiden (insbesondere Natrium oder Natriumverbindungen) oder Butyllithium bei Temperaturen zwischen 0 und 150° C umsetzt und dann das alkylierende Agens zufügt.

Anstellte der angeführten Alkylierungs-und Acylierungsmittel können auch andere in der Chemie gebräuchliche chemischäquivalente Mittel verwendet werden (siehe zum Beispiel auch L.F. und Mary Fieser "Reagents for Organic Synthesis", John Wiley and Sons, Inc., New York, 1967, Vol. 1, Seiten 1303-4 und Vol. 2, Seite 471).

In den verwendeten Ausgangsstoffen für das erfindungsgemäße Verfahren können vorhandene Hydroxygruppen, Mercaptogruppen, Carboxygruppen, Aminogruppen oder $C_1$-$C_6$-Alkylaminogruppen vorkommen, die durch übliche Schutzgruppen geschützt sind.

Es handelt sich hierbei um übliche Schutzgruppen, die durch Hydrolyse oder Hydrogenolyse leicht abspaltbar sind und während oder nach der Reaktion abgespalten werden. Es handelt sich hierbei um Schutzgruppen, die zum Beispiel in dem Buch von J.F.W. McOmie, Protective Groups in Organic Chemistry, Plenum Press, New York, 1973, Seiten 43 - 143 sowie 183 - 215 angegeben sind.

Falls solche Schutzgruppen bei der Verfahrensreaktion nicht abgespalten werden, dann erfolgt eine Abspaltung nach der Reaktion. Häufig enthalten die Ausgangsverbindungen aufgrund ihrer Herstellung bereits derartige Schutzgruppen.

Bei diesen Schutzgruppen handelt es sich beispielsweise um leicht solvolytisch abspaltbare Acylgruppen oder hydrierend abspaltbare Gruppen. Die solvolytisch abspaltbaren Schutzgruppen werden beispielsweise durch Verseifung mit verdünnten Säuren (zum Beispiel Esigsäure, Perchlorsäure, Salzsäure, Schwefelsäure, Ameisensäure, Trifluoressigsäure) oder mittels basischer Substanzen (Pottasche, Soda, wässrige

Alkalilösungen, alkoholische Alkalilösungen, $NH_3$) bei Temperaturen zwischen -50 und 150° C, insbesondere zwischen 0 und 100° C, abgespalten. Hydrierend abspaltbare Gruppen wie Arylalkylreste (Benzylrest) oder Aralkylcarbonylreste (Carbobenzoxyrest) werden zweckmäßig durch katalytische Hydrierung in Gegenwart üblicher Hydrierungskatalysatoren (Edelmetallkatalysatoren), insbesondere Palladium-Katalysatoren oder auch Platinkatalysatoren (Platinoxyd) oder Raney-Nickel, in einem Lösungs-oder Suspensionsmittel, gegebenenfalls unter erhöhtem Druck (zum Beispiel 1 - 50 bar) bei Temperaturen zwischen 20 - 150° C, insbesondere 30 - 100° C, vorzugsweise 40 - 80° C abgespalten. Als Lösungs-beziehungsweise Suspensionsmittel für die Abspaltung solcher Schutzgruppen kommen beispielsweise in Betracht: Wasser, niedere aliphatische Alkohole, cyclische Ether wie Dioxan oder Tetrahydrofuran, aliphatische Ether, Halogenkohlenwasserstoffe, Dimethylformamid und so weiter sowie Mischungen dieser Mittel. Als Schutzgruppen, die durch Hydrogenolyse abspaltbar sind, kommen beispielsweise in Frage: Benzylrest, α-Phenyläthylrest, im Benzolkern substituierte Benzylreste (p-Brom-oder p-Nitrobenzylrest), Carbobenzoxyrest beziehungsweise Carbobenzthiorest (wobei in solchen Resten der Benzolkern auch substituiert sein kann beispielsweise durch $NO_2$), tert.-Butyloxycarbonylrest. Beispiele für hydrolytische abspaltbare Reste sind: Phthalylrest, Tritylrest, p-Toluolsulfonylrest, tert.-Butyloxycarbonylrest, tert.-Butylrest, Dimethylmethylenrest und ähnliche sowie niedere Alkanoylreste wie Acetylrest, Propionlyrest, Trifluoracetylrest, Formylrest und ähnliche.
Falls die Ausgangsstoffe freie Carboxygruppen enthalten, ist es häufig zweckmäßig, diese vorher zu verestern, beispielsweise mit Benzylalkohol oder einem anderen niederen aliphatischen Alkohol (1 - 6, insbesondere 1 - 3 C-Atome). In den Endprodukten können derartige Estergruppen mittels Basen, beispielsweise alkoholischer Alkalilauge (zum Beispiel methanolische KOH) oder gegebenenfalls auch mittels Mineralsäuren wie Salzsäure oder Schwefelsäure in alkoholischer oder wässrig alkoholischer Lösung bei Temperaturen zwischen 20 und 100° C oder durch Hydrogenolyse abgespalten werden. Insbesondere kommen die bei der Peptid-Synthese üblichen Schutzgruppen und die dort üblichen Abspaltungsverfahren in Frage. Unter anderem wird hierzu auch auf das Buch von Jesse P. Greenstein und Milton Winitz "Chemistry of Amino Acids", New York, 1961, John Wiley and Sons, Inc., Volume 2, beispielsweise Seite 883 und folgende verwiesen. Auch die Carbalkoxygruppe (zum Beispiel niedrigmolekulare) kommt in Frage.

Die Überführung von Verbindungen der Formel I in die entsprechenden Amin-Oxide und/oder die Pyridin-N-oxide kann beispielsweise in inerten Lösungsmitteln wie Chloroform oder anderen Chlorkohlenwasserstoffen, Benzol, Toluol, Aceton, verdünnter Essigsäure oder Essigsäureäthylester mit Wasserstoffperoxyd, einer üblichen aliphatischen oder aromatischen Persäure (Peressigsäure, Benzopersäure, m-Chlorbenzopersäure) oder anderen Monosubstitutionsprodukten des Wasserstoffperoxids wie Alkaliperoxiden oder Alkylperoxiden (zum Beispiel tert.Butylperoxid) bei Temperaturen zwischen 0 und 150° C, vorzugsweise 0 bis 100° C, durchgeführt werden. Falls X = S ist, entstehen hierbei zuerst die entsprechenden Sulfoxide beziehungsweise Sulfone. Diese lassen sich dann jedoch weiter zu den Aminoxiden oxydieren.

Die Überführung von Verbindungen der Formel I worin X ein Schwefelatom bedeutet, in solche Verbindungen, worin X die Gruppe SO oder $SO_2$ ist, erfolgt ebenfalls durch Oxydation in an sich bekannter Weise. Als Oxydationsmittel können mit gutem Erfolg zum Beispiel Wasserstoffperoxid, Distickstofftetroxid, Kaliumpermanganat, Persäuren (zum Beispiel Benzopersäure, Phthalmonopersäure, Peressigsäure), Salpetersäure, Chromsäure oder andere bekannte Oxydationsmittel verwendet werden. Hierbei arbeitet man zweckmässig in Gegenwart von Wasser oder von Lösungsmitteln, zum Beispiel von Alkholen, Essigsäure (Eisessig), Essigsäureäthylester, Benzol, Aceton oder Chloroform. Besonders die niederen Alkohole, zum Beispiel Methanol oder auch Essigsäure sind gut geeignet, Bei der Oxydation mit 30 %igem Wasserstoffperoxid, Persäuren, Salpetersäure, nitrosen Gasen (Stickstoffdioxid) unter Kühlung, beispielsweise bei Temperaturen zwischen -20° C und +20° C erhält man im allgemeinen als Hauptprodukt das entsprechende Sulfoxid neben geringeren Mengen des Sulfons. Weiterhin können entsprechende Sulfoxide aus Verbindungen der Formel I, worin X = S ist, durch Oxydation mit Chromsäure (zum Beispiel in essigsaurer Lösung bei Temperaturen zwischen 50 - 100° C), durch Oxydation mit beispielsweise Jodosobenzol oder durch Behandlung mit Brom (zum Beispiel in einem Halogenkohlenwasserstoff wie Chloroform oder Tetrachlorkohlenstoff unter Kühlung) und anschließender Hydrolyse der Dibromderivate mittels Wasser oder verdünnter Alkalilauge hergestellt werden. Hinsichtlich der Reaktionsbedingungen sowie anderer Oxydationsmittel wird zum Beispiel auf Houben-Weyl, Methoden der Organischen Chemie, Band IX (1955), Seiten 211-218 verwiesen. Auch eine Oxydation von Sulfiden der Formel I (X = S) mit Dimethylsulfoxid bei höherer Temperatur (150 - 180° C) gemäß J.Org.Chem. 23 (1958), Seiten 2028-2029 ist möglich.
Die jeweils erhaltenen Sulfone und Sulfoxide können mittels üblicher Trennverfahren, beispielsweise durch Säulenchromatographie an Kieselgel getrennt werden.

Mit stärkeren Oxydationsmitteln wie zum Beispiel Kaliumpermanganat in essig-oder wäßrig--schwefelsaurer Lösung bei Temperaturen zwischen 50 und 100° C erhält man das entsprechende Sulfon in größerer Ausbeute beziehungsweise als Hauptprodukt. Die Oxydation von Verbindungen der Formel I worin

X = S oder SO ist, kann beispielsweise auch mittels Wasserstoffperoxid oder Persäuren bei höherer Temperatur, wie zum Beispiel 80 - 120° C (in essigsaurer Lösung oder in Eisessig und Essigsäureanhydrid, in Anwesenheit von Phosphorsäure oder einem sonstigen hierfür üblichen inerten Mittel), mittels Chromsäure, mittels anodischer Oxydation oder gegebenenfalls auch mittels Natriumhypochloritlösungen erfolgen (siehe Houben-Weyl, Methoden der Organischen Chemie, Band IX (1955), Seiten 227-231). Eine weitere Möglichkeit ist die Oxydation mit organischen Hydroperoxiden (zum Beispiel Alkylhydroperoxiden wie tert. Butylhydroperoxid) in Gegenwart von Vanadin-, Molybdän-oder Titanverbindungen (zum Beispiel Oxide der genannten Metalle wie Molybdändioxid, Vanadinpentoxid) in organischen Lösungsmitteln wie aromatischen Kohlenwasserstoffen (Benzol), Alkanolen (Ethanol) oder Estern aliphatischer Carbonsäuren mit Alkanolen (Ethylacetat) bei Temperaturen zwischen 40 - 120° C, vorzugsweise 50 - 80° C gemäß Angewandte Chemie 78 (1966), Seite 937.

Diejenigen Verbindungen der Formel I, die asymmetrische Kohlenstoffatome enthalten und in der Regel als Razemate anfallen, können in an sich bekannter Weise beispielsweise mit Hilfe einer optisch aktiven Säure in die optisch aktiven Isomeren gespalten werden. Es ist aber auch möglich, von vornherein eine optisch aktive Ausgangssubstanz einzusetzen, wobei dann als Endprodukt eine entsprechende optisch aktive beziehungsweise diastereomere Form erhalten wird. Die vorliegende Erfindung umfasst also auch die D-und L-Form wie auch die DL-Mischung für den Fall, daß in der Verbindung der Formel I ein asymmetrisches C-Atom vorkommt und für den Fall von zwei und mehr asymmetrischen C-Atomen ebenso die entsprechenden diastereomeren Formen.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern wieder in die Basen übergeführt werden. Von den letzteren lassen sich durch Umsetzung mit organischen oder anorganischen Säuren, insbesondere solchen, die zur Bildung von therapeutisch verwendbaren Salzen geeignet sind, Salze gewinnen.

Als therapeutisch verwendbare Salze kommen beispielsweise die Salze mit folgenden Säuren in Frage: Halogenwasserstoffsäuren (HCl, HBr), Schwefelsäure, Phosphorsäuren, Salpetersäure, Perchlorsäure, organische Mono-, Di-oder Tricarbonsäuren der aliphatischen, alicyclischen, aromatischen oder heterocyclischen Reihe sowie Sulfonsäuren. Beispiele hierfür sind: Ameisen-, Essig-, Propion-, Bernstein-, Glykol-, Milch-, Äpfel-, Wein-, Zitronen-, Ascorbin-, Malein-, Fumar-, Hydroxymalein-, Glucon-oder Brenztraubensäure; Phenylessigsäure, Benzoesäure, p-Aminosalicylsäure, Embonsäure, Methansulfon-, Ethansulfon-, oder Hydroxyethansulfonsäure; Ethylensulfonsäure; Halogenbenzolsulfon-, Toluolsulfon-oder Naphthalinsulfonsäure, Sulfanilsäure oder auch 8-Chlor-theophyllin.

Die erfindungsgemässen Verbindungen sind zur Herstellung pharmazeutischer Zusammensetzungen geeignet. Die pharmazeutischen Zusammensetzungen beziehungsweise Medikamente können eine oder mehrere der erfindungsgemässen Verbindungen enthalten. Zur Herstellung der pharmazeutischen Zubereitungen können die üblichen Träger-und Hilfsstoffe verwendet werden.

Zu dem Verfahren a):

Das Verfahren wird bei Temperaturen zwischen 0 und 150°C vorzugsweise 40 und 100°C in inerten Lösungsmitteln oder Suspensionsmitteln durchgeführt. Als Lösungs-oder Dispergiermittel kommen beispielsweise in Betracht: gegebenenfalls durch Chlor oder Brom substituierte aromatische Kohlenwasserstoffe wie zum Beispiel Benzol, Toluol, Xylol, Chlorbenzol, Pyridin; niedrigmolekulare aliphatische Ketone (zum Beispiel 3 - 6 C-Atome) wie zum Beispiel Aceton, Methylethylketon; halogenierte Kohlenwasserstoffe wie zum Beispiel Chloroform, Tetrachlorkohlenstoff, Chlorbenzol, Methylenchlorid; niedrigmolekulare aliphatische Ether (zum Beispiel 4 - 10 C-Atome) wie zum Beispiel Dimethoxyethan, Butylether; gesättigte cyclische Ether wie zum Beispiel Tetrahydrofuran, Dioxan; Sulfoxyde wie zum Beispiel Dimethylsulfoxid; tertiäre Säureamide wie zum Beispiel Dimethylformamid, Tetramethylharnstoff, N-Methylpyrrolidin, Hexamethylphosphorsäuretriamid; Acetonitril; niedrigmolekulare aliphatische Alkohole wie Methanol, Ethanol, Isopropanol, Amylalkohol, tert.-Butanol; cycloaliphatische Kohlenwasserstoffe wie Cyclohexan; niedrigmolekulare gesättigte Chlor-und Fluorkohlenwasserstoffe mit 1 - 5 C-Atomen, wobei die einzelnen C-Atome ein-oder mehrfach (2-bis 3-fach) durch Chlor und/oder Fluor substituiert sein können, wie Chloroform, Methylenchlorid. Auch wässrige Mischungen der genannten Lösungsmittel können verwendet werden. Häufig arbeitet man bei der Rückflußtemperatur der verwendeten Lösungs-beziehungsweise Dispergiermittel.

Gegebenenfalls kann man auch so vorgehen, daß man zuerst von der Verbindung II eine Alkaliverbindung (Natrium-, Kalium-oder auch Lithiumsalz zum Beispiel) herstellt, indem man sie in einem inerten

Lösungsmittel wie Dioxan, Dimethylformamid, Benzol oder Toluol mit einem Alkalimetall, Alkalihydrid oder Alkaliamiden (insbesondere Natrium oder Natirumverbindungen) oder Butyllithium bei Temperaturen zwischen 0 und 150° C reagieren läßt, und dann mit der Verbindung III (zum Beispiel in Form des Säurehalogenids) umsetzt.

Falls die freie Säure der Formel III verwendet wird, so ist deren Aktivierung durch die Gegenwart von Kondensationsmitteln wie Dicyclohexylcarbodiimid, Schwefligsäure-bis-alkylamiden (zum Beispiel $SO[N(CH_3)_2]_2$), N,N'-Carbonyldiimidazol und so weiter zweckmäßig (Organic Reactions, Vol. 12, 1962, Seiten 205 und 239).

Wird bei dem Verfahren a) eine Säure der Formel III mit aktivierter Carboxylgruppe eingesetzt, dann handelt es sich vorzugsweise um Verbindungen der allgemeinen Formel

$$R_3 - \underset{\underset{\displaystyle NR_4R_5}{|}}{CH} - COW \qquad VI$$

worin $R_3$, $R_4$ und $R_5$ die angegebenen Bedeutungen haben, und W ein Halogenatom, eine Gruppe der Formel -OR', -SR' oder eine Gruppe der Formel -OCO-R" bedeutet und R' einen $C_1$-$C_6$-Alkylrest oder im Falle von -OR' beziehungsweise -SR' auch einen Phenylrest, einen durch Nitrogruppen, $C_1$-$C_4$-Alkoxygruppen, $C_1$-$C_4$-Alkylgruppen oder Halogenatome (Chlor, Fluor, Brom) substituierten Phenylrest, einen Cyanmethylrest oder einen Carboxymethylrest und R" einen geraden oder verzweigten $C_1$-$C_6$-Alkylrest, einen $C_1$-$C_6$-Alkoxyrest, einen Phenoxyrest oder einen Carbobenzoxyrest oder auch den $R_3$-$CH(NR_4R_5)$-darstellt.

Falls W ein Halogenatom bedeutet, handelt es sich vorzugsweise um Chlor, Brom oder Jod; falls R' beziehungsweise R" Alkylreste oder Alkoxyreste bedeuten, dann sind diese vorzugsweise niedermolekular und bestehen aus 1-4 Kohlenstoffatomen.

Häufig, insbesondere wenn W (Formel VI) ein Halogenatom oder die Gruppe -OCOR" bedeutet, ist die Gegenwart von säurebindenden Stoffen wie Alkalihydroxiden, Alkalicarbonaten, Alkalihydrogencarbonaten, Alkaliacetaten, Erdalkalicarbonaten, Trialkylaminen, Dialkylaminen, Pyridin und ähnlichen oder auch überschüssige Verbindung II zweckmäßig. Dabei kann das säurebindende Agens auch gleichzeitig allein oder im Gemisch mit anderen üblichen Mitteln als Lösungsmittel benutzt werden (zum Beispiel Pyridin). Es ist darauf zu achten, daß nichtumgesetzte Ausgangssubstanz III beziehungsweise VI, insbesondere falls es sich um ein Säurechlorid handelt, sorgfältig neutralisiert und entfernt wird. Häufig empfiehlt sich eine chromatographische Reinigung des Reaktionsproduktes über Kieselgel, wobei zum Beispiel mit einer Chloroform-Ethanol-Mischung (Ethanolgehalt zum Beispiel 4 %) gegebenenfalls unter Zusatz von wässrigem Ammoniak eluiert wird.

Anstelle der angeführten Aktivierungsmittel für die Carboxylgruppe können auch andere in der Chemie gebräuchliche chemisch-äquivalente Mittel verwendet werden. Derartige Mittel und Verfahren sind zum Beispiel angegeben in folgenden Literaturstellen: L.F. und Mary Fieser "Reagents for Organic Synthesis", John Wiley and Sons, Inc., New York, 1967, Vol. 1, Seiten 1303-4 and Vol. 2, Seite 471); Jakubke, Jeschkeit, "Aminosäuren, Peptide, Proteine", Akademie-Verlag, Berlin, 1982, 2. Auflage, Seiten 158 - 183. Die zuvor genannten Literaturstellen und deren Inhalt gemäß den angegebenen Seitenzahlen sind Bestandteil der Anmeldung.

Zu dem Verfahren b):

Das Verfahren zur Herstellung von Verbindungen der Formel I aus Verbindungen der Formel IV und V wird in einem Lösungs-oder Dispergiermittel bei Temperaturen zwischen 20 und 200° C, vorzugsweise 40 und 150° C, insbesondere 50 und 120° C durchgeführt. Als Lösungs-beziehungsweise Dispergiermittel kommen zum Beispiel in Frage: niedere aliphatische Alkohole (1-6 C-Atome); Propanol, Isopropanol, Butanol, niedere aliphatische Ether (Diethylether, Diisopropylether), aromatische Kohlenwasserstoffe (Benzol, Toluol, Xylol), cyclische Ether (Dioxan, Tetrahydrofuran), Ester von niederen aliphatischen Carbonsäuren mit niederen aliphatischen Alkoholen, Amide und N-alkylsubstituierte Amide von aliphatischen $C_1$-$C_4$-Carbonsäuren (Dimethylformamid) Dimethylacetamid), $C_1$-$C_6$-Dialkylsulfone (Dimethylsulfon, Tetramethylensulfon), $C_1$-$C_6$-Dialkylsulfoxide (Dimethylsulfoxid) sowie weitere aprotische Mittel wie N-Methylpyrrolidon, Tetramethylharnstoff, Hexamethylphosphorsäuretriamid, Acetonitril. Die einzelnen Alylreste der oben

angegebenen Lösungsmittel enthalten beispielsweise 1-6, insbesondere 1-4 Kohlenstoffatome.
Das Verfahren wird zweckmäßig in Gegenwart von Kondensationsmitteln durchgeführt. Als derartige Kondensationsmittel kommen zum Beispiel in Frage: anorganische Kondensationsmittel wie Alkali-oder Erdalkalihydroxide, Alkalihydride, Alkaliamide, Alkali-oder Erdalkali-carbonate oder organische Basen wie Pyridin, tertiäre Amine, Piperidin, Alkalialkoholate, Alkaliacetate oder auch Triethylphosphat. Bei den Alkalimetallen handelt es sich insbesondere um Natrium oder Kalium. Es kann auch unter Phasen-Transfer-Bedingungen (das heißt unter Zusatz eines oder mehrerer langkettiger Amine wie einem Benzyltributyl-ammonium-halogenid, einem Tetrabutyl-ammonium-halogenid oder Benzyl-triphenyl-phosphoniumchlorid) gearbeitet werden.
Die Ausgangsstoffe der Formeln IV und V können auch in Form ihrer Salze eingesetzt werden.
Im allgemeinen stellt man aus der Ausgangskomponente, die die Hydroxy-beziehungsweise Mercaptogruppe enthält, zuerst unter Verwendung einer wie oben angegebenen Alkaliverbindung das entsprechende Salz her und setzt dieses dann anschliessend mit der zweiten Reaktionskomponente um. Vorzugsweise sind in der Ausganskomponente der Formel V die Aminogruppe sowie gegebenenfalls weitere vorhandene Amino-, Hydroxy-und/oder Mercaptogruppen durch übliche Schutzgruppen geschützt, wobei solche Schutzgruppen nach Beendigung der Reaktion leicht solvolytisch oder hydrierend abspaltbar sind.

Falls Y der Formel V eine $C_1$-$C_6$-Alkyl-sulfonyloxygruppe ist, handelt es sich vorzugsweise um eine solche mit 1 - 4 C-Atomen im Alkylteil (beispielsweise die Methylsulfonyloxygruppe). Falls Y der Formel V eine Arylsulfonyloxygruppe ist, handelt es sich bei dem Arylrest vorzugsweise um einen Phenyl-oder Naphthylrest, wobei diese gegebenenfalls durch $C_1$-$C_4$-Alkylreste (insbesondere Methylreste) substituiert sein können (zum Beispiel p-Toluolsulfonyloxygruppe).

Ausgangsstoffe der Formel V können aus den bekannten Verbindungen der Formel V, worin die Gruppe $R_3$-CH($NR_4R_5$)-CO-Wasserstoff ist, durch Einführung des zuvor genannten Restes durch N-Acylierung in an sich bekannter Weise beziehungsweise gemäß Bedingungen erhalten werden, die in dieser Anmeldung für das Verfahren a) angegeben sind.

Aus Verbindungen der Formel V, worin Y die Hydroxygruppe ist, können Ausgangsstoffe der Formel V erhalten werden, worin Y ein Halogenatom ist und zwar beispielsweise durch Umsetzung mit Thionylhalogeniden (Chloriden, Bromiden, Jodiden) - oder Sulfonsäurechloriden in Halogenkohlenwasserstoffen (Chloroform) oder aromatischen Kohlenswasserstoffen (Benzol) oder in Pyridin bei Temperaturen zwischen 20 und 150° C (vorzugsweise Siedetemperatur des verwendeten Lösungsmittels). Ausgangsstoffe der Formel V, worin Y eine Alkylsulfonyloxygruppe oder eine Arylsulfonyloxygruppe ist, können zum Beispiel aus den entsprechenden Hydroxyverbindungen (Y = OH) durch Umsetzung mit $C_1$-$C_6$-Alkyl-sulfonsäurechloriden oder den entsprechenden Arylsulfonsäurechloriden in hierfür üblichen inerten Lösungsmitteln (Benzol, Toluol, Xylol, Chloroform, Methylenchlorid, Dioxan) bei Temperaturen zwischen 20 - 150° C erhalten werden. Zweckmäßig wird hierbei in Gegenwart einer säurebindenden Substanz (zum Beispiel tertiäre Amine wie Triethylamin) gearbeitet.
In all diesen Fällen werden die vorhandenen Aminogruppen sowie gegebenenfalls weitere vorhandene Hydroxy-oder Mercaptogruppen durch leicht abspaltbare Schutzgruppen geschützt.

Aus den Halogeniden der Formel V (Y = Halogen) können beispielsweise durch Umsetzung mit Alkalisulfiden Ausgangsstoffe der Formel V erhalten werden, worin Y die Mecaptogruppe ist. Diese Umsetzungen können analog C.Ferri, Reaktionnen der organischen Synthese 1978, Seiten 205 - 209 oder analog der DE-OS 2 230 392 zum Beispiel Seite 9 erfolgen.

Ausgangsstoffe der Formel V mit folgender Struktur

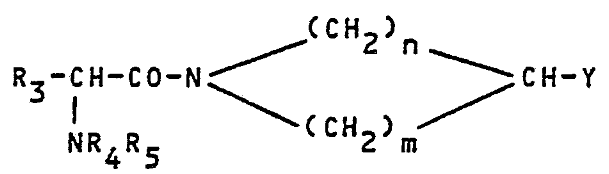

Va

(Y = OH, Halogen, SH)

können zum Beispiel wie folgt erhalten werden:
Eine Verbindung der Formel Va, worin die Gruppierung >CHY die Struktur >C=O hat, wird analog H. Barrera und R.E. Lyle, J. Org. Chem., 27 (1962), Seiten 641-643 mit Schwefelwasserstoff umgesetzt und anschließend mit Natriumborhydrid zur Verbindung Va, worin Y = SH ist, reduziert.

Mann kann aber auch in einer Verbindung Va ( >CHY=CO) die Ketogruppe in bekannter Weise mit Alkaliboranaten (Na, K, Li) oder anderen komplexen Metallhydriden (zum Beispiel Lithiumaluminiumhydrid) zur Hydroxygruppe reduzieren (siehe Houben-Weyl, Methoden der Organischen Chemie, Band 4/1d, 1981, Seite 271 ff.), die Hydroxygruppe mittels üblicher Chlorierungsmittel (zum Beispiel Thionylchlorid, Sulfurylchlorid) gegen ein Chloratom austauschen (siehe Houben-Weyl, Methoden der Organischen Chemie, Band 5/3, 1962, Seiten 862-912), aus dem so erhaltenen Chlorid mit Magnesium die entsprechende Grignard-Verbindung (Formel Va Y=MgCl) herstellen (siehe Houben-Weyl, Methoden der Organischen Chemie, Band 13/2a, 1973, Seiten 53-85) und aus einer solchen Grignard-Verbindung mittels Schwefel oder Thionylchlorid (siehe Houben-Weyl, Methoden der Organischen Chemie, Band 9, 1955, Seite 19; E.E. Reid, Organic Chemistry of Bivalent Sulfur Bd. I Chem.Publ.Corp., New York, 1958, Seite 37) die Mercapto-Verbindung Va, worin Y = SH ist, herstellen. Auch hier werden vorhandene Aminogruppen sowie gegebenenfalls vorhandene Carboxygruppen, Hydroxygruppen oder Mercaptogruppen durch entsprechende Schutzgruppen geschützt.

Weitere pharmakologische und pharmazeutische Angaben

Die erfindungsgemäßen Verbindungen besitzen eine gute antinociceptive Wirkung (Elektroschmerz-Test der Maus) Beispielsweise wird bei oben genannter Versuchsmethode bei einer oralen Dosis von 10 mg/kg Körpergewicht Maus eine deutliche antinociceptive Wirkung festgestellt.

Als niedrigste, bereits antinociceptiv wirksame Dosis in dem oben angegebenen Tierversuch ist beispielsweise

2,5 mg/kg oral
1,25 mg/kg intraperitoneal
0,25 mg/kg intravenös

Als allgemeiner Dosisbereich für die antinociceptive Wirkung (Tierversuch wie oben) kommt beispielsweise in Frage:
2,5 - 40 mg/kg oral, insbesondere 5 - 20
1,25 - 30 mg/kg intraperitoneal, insbesondere 2,5 - 15
0,25 - 5 mg/kg intravenös, insbesondere 0,5 - 2

Die antinociceptive Wirkungsrichtung der erfindungsgemäßen Verbindungen ist zum Beispiel mit der Wirkung von Morphin vergleichbar, jedoch bestehen hierzu insbesondere folgende Unterschiede: kein Opiat, deshalb kein Sucht-und Abhängigkeitspotential vom Opiattyp.
Die erfindungsgemäßen Verbindungen stellen wirksame Analgetika dar.

Die pharmazeutischen Zubereitungen für die antinociceptive Wirkung enthalten im allgemeinen zwischen 2,5 bis 30 vorzugsweise 5 bis 20 mg der erfindungsgemäßen aktiven Komponente.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen.

Die Einzeldosis der erfindungsgemäßen aktiven Komponenten kann beispielsweise liegen

a) bei oralen Arzneiformen zwischen 2,5 - 30, vorzugsweise 5 - 20 mg

b) bei parenteralen Arzneiformen (zum Beispiel intravenös, intramuskulär) zwischen 0,5 - 6, vorzugsweise 1,0 - 3,0 mg.

- (Die Dosen sind jeweils bezogen auf die freie Base)

Beispielsweise können 3mal täglich 1 bis 3 Tabletten mit einem Gehalt von 5 bis 20 mg wirksamer Substanz oder zum Beispiel bei intravenöser Injektion 1 bis 3mal täglich eine Ampulle von 2 bis 4 ml Inhalt mit 1 bis 4 mg Substanz empfohlen werden. Bei oraler Verabreichung ist die minimale tägliche Dosis beispielsweise 10 mg; die maximale tägliche Dosis bei oraler Verabreichung für die antinociceptive Wirkung soll nicht über 600 mg liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 200 und 300 mg/kg (beziehungsweise oberhalb 250 mg/kg).

Die Arzneimittel können in der Humanmedizin, der Veterinärmedizin sowie in der Landwirtschaft allein oder im Gemisch mit anderen pharmakologisch aktiven Stoffen verwendet werden.

Darüber hinaus besitzen die erfindungsgemäßen Verbindungen auch eine gute antidepressive Wirkung (Schwimm-Test nach Porsolt).
Beispielsweise wird bei oben genannter Versuchsmethode bei einer Dosis von 20 mg/kg Körpergewicht Ratte (intraperitoneale Applikation) eine 50 - 80%ige Wirkung erzielt.

Die niedrigste bereits antidepressiv wirksame Dosis in dem oben angegebenen Tierversuch ist beispielsweise

10 mg/kg oral
5 mg/kg intraperitoneal
1 mg/kg intravenös

Als allgemeiner Dosisbereich für die antidepressive Wirkung (Tierversuch wie oben) kommt beispielsweise in Frage:

10 - 60 mg/kg oral, insbesondere 20 - 40 mg
5 - 40 mg/kg intraperitoneal, insbesondere 10 - 30 mg
1 - 15 mg/kg intravenös, insbesondere 3 - 12 mg

Die antidepressive Wirkungsrichtung der erfindungsgemäßen Verbindungen ist mit der Wirkung des bekannten Arzneimittelwirkstoffs Imipramin vergleichbar, jedoch bestehen hierzu insbesondere folgende Unterschiede: länger anhaltende Wirkung, stärkere Wirkung, mit überwiegend stimulierender (thymoretischer) Wirkung. Die erfindungsgemäßen Verbindungen stellen daher auch wirksame Antidepressiva dar.

Die pharmazeutischen Zubereitungen für die antidepressive Wirkung enthalten im allgemeinen zwischen 1 bis 200 vorzugsweise 10 bis 100 mg der erfindungsgemäßen aktiven Komponente.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen.
Bevorzugte Anwendungsformen sind Tabletten, die zwischen 20 und 100 mg oder Lösungen, die zwischen 0,1 bis 0,5 Gewichtsprozent an aktiver Substanz enthalten.

Die Einzeldosis der erfindungsgemäßen aktiven Komponenten für die antidepressive Wirkung kann beispielsweise liegen

a) bei oralen Arzneiformen zwischen 20 - 100 mg
b) bei parenteralen Arzneiformen (zum Beispiel intravenös, intramuskulär) zwischen 1 - 10 mg
- (Die Dosen sind jeweils bezogen auf die freie Base)

Beispielsweise können 3mal täglich 1 bis 3 Tabletten mit einem Gehalt von 20 bis 200 mg wirksamer Substanz oder zum Beispiel bei intravenöser Injektion 1 bis 3mal täglich eine Ampulle von 2 bis 4 ml Inhalt mit 2 bis 5 mg Substanz empfohlen werden. Bei oraler Verabreichung ist die minimale tägliche Dosis beispielsweise 20 mg; die maximale tägliche Dosis bei oraler Verabreichung für die antidepressive Wirkung soll nicht über 600 mg liegen.

Beispiel 1

[N-Glycyl-piperidyl-(4)]-[6-chlor-pyridyl-(2)-]-sulfid

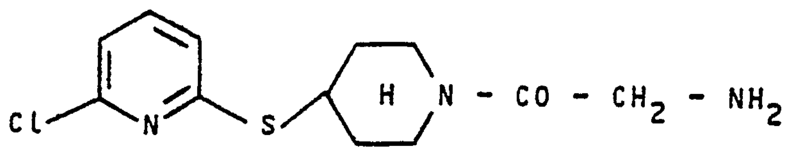

Zu einer Lösung von 12,3 g (0,07 Mol) N-tert.-Butyloxycarbonylglycin in 80 ml Dioxan werden unter Rühren bei Raumtemperatur 11,350 g (0,07 Mol) 1,1-Carbonyldiimidazol auf einmal zugegeben.
Unter lebhafter $CO_2$-Entwicklung setzt die Reaktion ein. Zur Vervollständigung wird noch 30 Minuten bei 40° C erwärmt. Nach Abkühlen auf 20° C wird eine Lösung von 14,3 g (0,0624 Mol) Piperidyl-(4)-[6-chlor-pyridyl-(2)]-sulfid, in 20 ml Dioxan gelöst, zugefügt. Die Temperatur steigt auf 35° C an. Es wird weitere 3 Stunden bei 45-50° C erhitzt. Danach wird das Lösungsmittel im Rotationsverdampfer entfernt, der ölige Rückstand in 200 ml Ether gelöst und die Lösung 2mal mit je 50 ml Wasser ausgeschüttelt. Die Etherphase wird mit $MgSO_4$ getrocknet, filtriert und eingeengt. Der Rückstand wird in 60 ml Isopropanol gelöst und die Lösung mit isopropanolischer Salzsäure angesäuert. Danach wird etwa 4 Stunden unter Rühren und unter Rückfluß erhitzt. Dann wird die Lösung eingeengt, der Rückstand in Ethanol gelöst und mit Ether bis zur beginnenden Trübung versetzt. Nach 36stündigem Stehen im verschlossenen Gefäß bei Raumtemperatur wird das Kristallisat abgesaugt, mehrmals mit einer Mischung aus gleichen Volumteilen Ethanol und Ether gewaschen und getrocknet.
Schmelzpunkt des Hydrochlorids: 146-148° C
Ausbeute: 7,6 g.

Beispiel 2

(L)-[N-Alanyl-piperidyl-(4)]-[6-chlor-pyridyl-(2)-]-sulfid

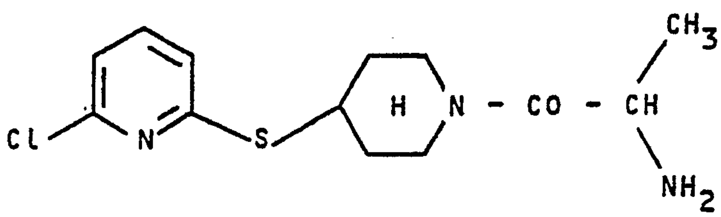

17,88 g L-tert.-Butyloxycarbonylalanin, gelöst in 150 ml Dioxan, werden mit 15,3 g 1,1-Carbonyldiimidazol versetzt. Die Reaktion setzt unter lebhafter $CO_2$-Entwicklung ein und wird durch 30minütiges Erwärmen auf 40° C vervollständigt. Es wird wieder auf Raumtemperatur abgekühlt. Nach Zufügen einer Lösung von 21,6 g Piperidyl-(4)-[6-chlor-pyridyl-(2)-]-sulfid in 30 ml Dioxan wird für 3 Stunden auf 45-50° C erwärmt. Danach wird das Lösungsmittel im Rotationsverdampfer entfernt, der Rückstand in 300 ml Ether gelöst und die Lösung 2mal mit je 70 ml Wasser ausgeschüttelt. Die Etherphase wird mit $MgSO_4$ getrocknet, filtriert und eingeengt. Der Rückstand wird in 100 ml Isopropanol gelöst und die Lösung mit isopropanolischer Salzsäure angesäuert. Nach vierstündigem Erhitzen unter Rückfluß wird die Lösung weitgehend eingeengt. Der Rückstand wird in Ethanol gelöst, filtriert und mit Ether bis zur beginnenden Trübung versetzt. Die Substanz kristallisiert spontan. Der kristalline Niederschlag wird abgesaugt, mit einer Mischung aus gleichen Volumenteilen Ethanol und Ether gewaschen und getrocknet.
Das Hydrochlorid schmilzt bei 195-196° C
Ausbeute: 20,3 g ( 64% der Theorie).

Analog den vorangegangenen Beispielen werden die Verbindungen gemäß der Tabelle 1 hergestellt. Es handelt sich hierbei um Verbindungen der folgenden Formel

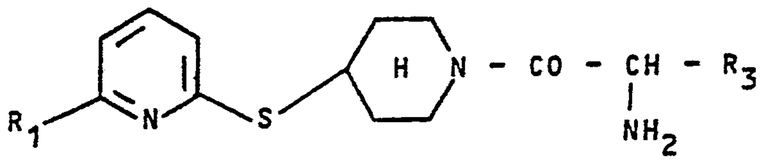

Es werden jeweils 0,03 Mol der geschützten Aminosäure (zum Beispiel N-tert.-Butyloxycarbonyl-aminosäure) mit 0,025 Mol Piperidyl-(4)-[6-chlor-pyridyl-(2)]-sulfid in Dioxan umgesetzt. Gegebenenfalls wird vor dem Aufnehmen des Reaktionsproduktes in Ether (das heißt nach Entfernen des Dioxans) mit Wasser versetzt (zum Beispiel 50 ml Wasser).

Bei Beispiel 8 wird die Reaktionslösung mit 80 ml Wasser versetzt, nach Abtrennen der Methylenchlorid-Phase nochmals mit $CH_2Cl_2$ ausgeschüttelt, die organische Phase getrocknet und das Lösungsmittel entfernt. Anschließend erfolgt Abspaltung der tert.-Butyloxycarbonyl-Schutzgruppe mit Salzsäure in Isopropanol.

Die Abspaltung der Aminosäureschutzgruppe erfolgt jeweils durch Erhitzen mit Salzsäure in Isopropanol; Dauer 2 Stunden (bei Beispiel 3 = 3 Stunden). Bei den Beispielen 3 und 5 erfolgt die Isolierung des Reaktionsproduktes in Form de Maleats. Das Maleat wird mittels Maleinsäure in Aceton-Lösung hergestellt und durch Zusatz von Diethylether abgeschieden.

Tabelle 1

| Beispiel-Nr. | $R_1$ | $R_3$ | Schmelzpunkt des HCl-Salzes; °C | Aminosäure-Ausgangsverbindung | Reaktionsmedium | Ausbeute |
|---|---|---|---|---|---|---|
| 3 | Cl | $-CH(CH_3)_2$ | 160-161 (Maleat) | BOC-L-Valin | Dioxan | 6 g |
| 4 | Cl | $-CH_2-CH(CH_3)_2$ | 84 | BOC-L-Leucin (Hydrat) | Dioxan | 8,5 g |
| 5 | Cl | $-(CH_2)_3-CH_3$ | 149-150 (Maleat) | BOC- -Norleucin | Dioxan | 6 g |
| 6 | Cl | $-C_6H_5$ | 240-242 Z | BOC-Phenylglycin | Dioxan | 8 g |
| 7 | Cl | $-CH_2-C_6H_5$ | 120 | BOC- -Phenylalanin | Dioxan | 7,4 g |
| 8 | Cl | $-(CH_2)_2-SCH_3$ | 92 | BOC-L-Methionin | $CH_2Cl_2$ (60 ml) | 2,5 g |
| 9 | Cl | $-C(SH)(CH_3)_2$ | 144 | BOC-D-Penicillamin | Dioxan | 4,5 g |

Z = Zersetzung; BOC = tert.-Butyloxycarbonyl

Beispiele für pharmazeutische Zubereitungen

Kapseln mit 100 mg Wirkstoff

10 kg Wirksubstanz (Verbindung gemäß Beispiel 1 als Hydrochlorid) werden in einer Wirbelschicht-Sprühgranulationsapparatur mit einer Lösung aus 0,25 kg Gelatine in 2,25 kg Wasser in bekannter Weise granuliert. Nach Zumischen von 0,80 kg Maisstärke, 0,1 kg. Magnesiumstearat und 0,05 kg hochdispersem Siliciumdioxid wird die Mischung in einer Füllmenge von jeweils 112 mg in Hartgelatinekapseln der Größe 3 gefüllt. Eine Kapsel enthält 100 mg Wirksustanz.

Beispiel für Ampullen

20 g Wirksubstanz (Verbindung gemäß Beispiel 1 als Hydrochlorid) werden zusammen mit 14,35 g Natriumchlorid in 1,9 Liter Wasser für Injektionszwecke gelöst. Die Lösung wird mit Wasser für Injektionszwecke auf 2 Liter aufgefüllt, durch ein Membranfilter der Porenweite 0,2 um filtriert und unter sterilen Bedingungen zu 2,15 ml in sterile Ampullen gefüllt. Eine Ampulle enthält 10 mg Wirksubstanz in 2 ml Lösung.

**Ansprüche**

1. Verbindungen der Formel

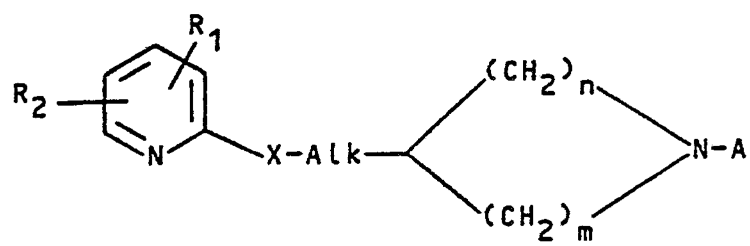

I

worin die Reste $R_1$ und $R_2$ gleich oder verschieden sind und Wasserstoff, Halogenatome, eine Trifluormethylgruppe, eine Cyanogruppe, eine Nitrogruppe, eine Aminogruppe, eine Mono-$C_1$-$C_6$-alkylaminogruppe, eine Di-$C_1$-$C_6$-alkylaminogruppe, eine Aminogruppe, die durch einen Phenyl-$C_1$-$C_4$-alkylrest oder einen Halogenphenyl-$C_1$-$C_4$-alkylrest substituiert ist, eine $C_2$-$C_6$-Alkanoylaminogruppe, eine $C_1$-$C_6$-Alkoxy carbonylaminogruppe, eine gegebenenfalls durch einen Phenylrest substituierte $C_1$-$C_6$-Alkylgruppe, eine Hydroxygruppe, eine $C_1$-$C_6$-Alkoxygruppe, eine $C_2$-$C_6$-Alkanoyloxygruppe, eine Phenoxygruppe oder eine gegebenenfalls durch eine oder zwei $C_1$-$C_6$-Alkylgruppen substituierte Carbamoylgruppe bedeuten, der Rest A die Gruppe

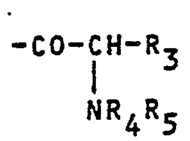

darstellt, worin $R_3$ Wasserstoff, einen Phenylrest, einen Indolyl-(3)-methylrest, Imidazolyl-(4)-methylrest, eine $C_1$-$C_{10}$-Alkylgruppe bedeutet, oder worin $R_3$ eine $C_1$-$C_{10}$-Alkylgruppe bedeutet, welche durch eine Carboxygruppe, eine $C_1$-$C_6$-Alkoxycarbonylgruppe, eine Aminocarbonylgruppe, eine Hydroxygruppe, eine $C_1$-$C_6$-Alkoxygruppe, eine $C_2$-$C_6$-Alkanoyloxygruppe, eine Mercaptogruppe, eine $C_1$-$C_6$-Alkylthiogruppe,

eine $C_2$-$C_6$-Alkanoylmercaptogruppe, eine Phenylgruppe, eine Hydroxyphenylgruppe, eine Dihydroxyphenylgruppe, eine Amino-$C_1$-$C_6$-alkylthiogruppe, eine Amino-$C_1$-$C_6$-alkyloxygruppe, eine Aminogruppe, eine Ureidogruppe ($H_2NCONH$-) oder eine Guanidinogruppe substituiert ist, oder worin $R_3$ zusammen mit dem Strukturteil $>CH(NHR_4)$ den Pyrrolidin-2-yl-rest (Prolinrest) oder den 4-Hydroxy-pyrrolidin-2-yl-rest darstellt, $R_4$ Wasserstoff, Benzyl oder einen $C_1$-$C_6$-Alkylrest, $R_5$ Wasserstoff, Benzyl, einen $C_1$-$C_6$-Alkylrest, einen $C_2$-$C_6$-Alkanoylrest oder die Gruppe

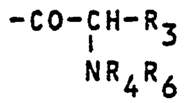

bedeutet, wobei $R_3$ und $R_4$ die bereits angegebenen Bedeutungen haben und $R_6$ Wasserstoff, Benzyl oder $C_2$-$C_6$-Alkanoyl ist, X Sauerstoff, Schwefel, SO oder $SO_2$ bedeutet, Alk Alkylen mit 0-4 C-Atomen ist und n und m gleich oder verschieden sind und die Zahlen 1-3 annehmen können, wobei n auch 0 sein kann, wenn Alk Alkylen mit mindestens einem Kohlenstoffatom ist und m für diesen Fall die Zahlen 2-6 annimmt, deren Pyridin-N-oxide und/oder Aminoxide und deren physiologisch verträglichen Salze.

2. Verfahren zur Herstellung von Verbindungen der Formel

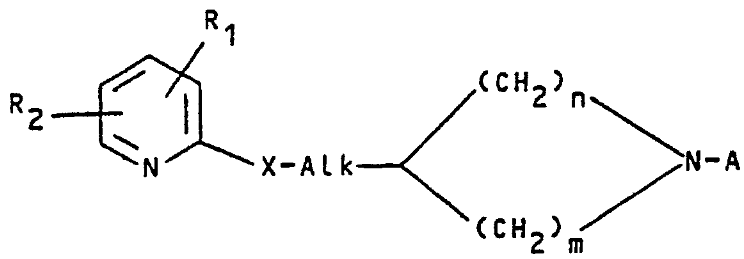

I

worin die Reste $R_1$ und $R_2$ gleich oder verschieden sind und Wasserstoff, Halogenatome, eine Trifluormethylgruppe, eine Cyanogruppe, eine Nitrogruppe, eine Aminogruppe, eine Mono-$C_1$-$C_6$-alkylaminogruppe, eine Di-$C_1$-$C_6$-alkylaminogruppe, eine Aminogruppe, die durch einen Phenyl-$C_1$-$C_4$-alkylrest oder einen Halogenphenyl-$C_1$-$C_4$-alkylrest substituiert ist, eine $C_1$-$C_6$-Alkanoylaminogruppe, eine $C_1$-$C_6$-Alkoxy carbonylaminogruppe, eine gegebenenfalls durch einen Phenylrest substituierte $C_1$-$C_6$-Alkylgruppe, eine Hydroxygruppe, eine $C_1$-$C_6$-Alkoxygruppe, eine $C_2$-$C_6$-Alkanoyloxygruppe, eine Phenoxygruppe oder eine gegebenenfalls durch eine oder zwei $C_1$-$C_6$-Alkylgruppen substituierte Carbamoylgruppe bedeuten, der Rest A die Gruppe

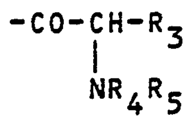

darstellt, worin $R_3$ Wasserstoff, einen Phenylrest, einen-Indolyl-(3)-methylrest, Imidazolyl-(4)-methylrest, eine $C_1$-$C_{10}$-Alkylgruppe bedeutet, oder worin $R_3$ eine $C_1$-$C_{10}$-Alkylgruppe bedeutet, welche durch eine Carboxygruppe, eine $C_1$-$C_6$-Alkoxycarbonylgruppe, eine Aminocarbonylgruppe, eine Hydroxygruppe, eine $C_1$-$C_6$-Alkoxygruppe, eine $C_2$-$C_6$-Alkanoyloxygruppe, eine Mecaptogruppe, eine $C_1$-$C_6$-Alkylthiogruppe, eine $C_2$-$C_6$-Alkanoylmercaptogruppe, eine Phenylgruppe, eine Hydroxyphenylgruppe, eine Dihydroxyphenylgruppe, eine Amino-$C_1$-$C_6$-alkylthiogruppe, eine Amino-$C_1$-$C_6$-alkyloxygruppe, eine Aminogruppe, eine Ureidogruppe ($H_2NCONH$-) oder eine Guanidinogruppe substituiert ist, oder worin $R_3$ zusammen mit dem Strukturteil $>CH(NHR_4)$ den Pyrrolidin-2-yl-rest (Prolinrest) oder den 4-Hydroxy-pyrrolidin-2-yl-rest darstellt, $R_4$ Wasserstoff, Benzyl oder einen $C_1$-$C_6$-Alkylrest, $R_5$ Wasserstoff, Benzyl, einen $C_1$-$C_6$-Alkylrest, einen $C_2$-$C_6$-Alkanoylrest oder die Gruppe

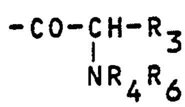

bedeutet, wobei $R_3$ und $R_4$ die bereits angegebenen Bedeutungen haben und $R_6$ Wasserstoff, Benzyl oder $C_2$-$C_6$-Alkanoyl ist, X Sauerstoff, Schwefel, SO oder $SO_2$ bedeutet, Alk Alkylen mit 0-4 C-Atomen ist und n und m gleich oder verschieden sind und die Zahlen 1-3 annehmen können, wobei n auch 0 sein kann, wenn Alk Alkylen mit mindestens einem Kohlenstoffatom ist und m für diesen Fall die Zahlen 2-6 annimmt, deren Pyridin-N-oxiden und/oder Aminoxiden und deren physiologisch verträglichen Salzen,
dadurch gekennzeichnet,
daß man

a) eine Verbindung der allgemeinen Formel

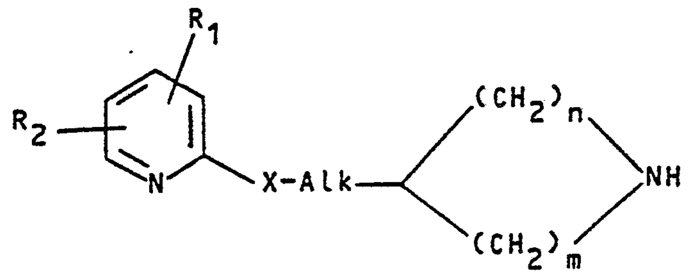

II

worin $R_1$, $R_2$, X, Alk, n und m die angegebenen Bedeutungen haben, mit einer Säure der allgemeinen Formel

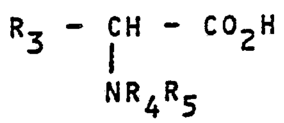

III

worin $R_3$, $R_4$ und $R_5$ die angegebenen Bedeutungen haben, wobei die Carboxygruppe auch aktiviert sein kann, umsetzt, wobei in den Ausgangs-Verbindungen II und III vorhandene primäre oder sekundäre Aminogruppen, Carboxygruppen, Hydroxygruppen und/oder Mercaptogruppen durch übliche Schutzgruppen geschützt sein können, in den erhaltenen Verbindungen gegebenenfalls vorhandene Schutzgruppen abspaltet, gegebenenfalls vorhandene freie Hydroxygruppen, Mercaptogruppen, primäre oder sekundäre Aminogruppen alkyliert oder acyliert und/oder Verbindungen der Formel I in die entsprechenden Sulfone, Sulfoxide, Aminoxide oder Pyridin-N-oxide überführt, oder

b) eine Verbindung der allgemeinen Formel

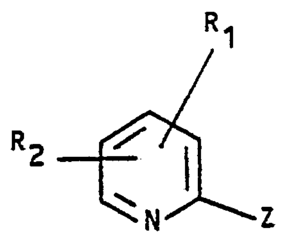

IV

oder deren Pyridin-N-oxid, worin $R_1$ und $R_2$ die angegebenen Bedeutungen haben, mit einer Verbindung der allgemeinen Formel

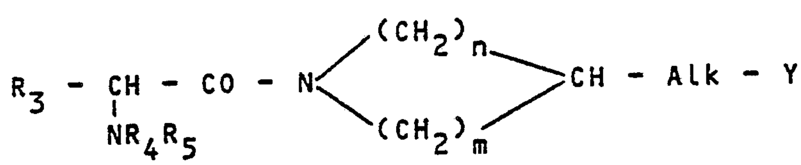

V

umsetzt, wobei in der Formel V die Symbole $R_3$, $R_4$, $R_5$, n, m und Alk die angegebenen Bedeutungen haben, und in den Ausgangsverbindungen IV und V vorhandene primäre oder sekundäre Aminogruppen,

Carboxygruppen, Hydroxygruppen und/oder Mercaptogruppen durch übliche Schutzgruppen geschützt sein können, und Y ein Halogenatom, eine $C_1$-$C_6$-Alkyl-sulfonyloxygruppe oder eine Aryl-sulfonyloxygruppe ist, falls Z der Formel IV eine Hydroxygruppe oder eine Mercaptogruppe ist, oder worin Y eine Hydroxygruppe oder eine Mercaptogruppe bedeutet, falls Z der Formel IV ein Halogenatom ist, in den erhaltenen Verbindungen gegebenenfalls vorhandene Schutzgruppen abspaltet, gegebenenfalls vorhandene freie Hydroxygruppen, Mercaptogruppen, primäre oder sekundäre Aminogruppen alkyliert oder acyliert, und/oder Verbindungen der Formel I in die entsprechenden Sulfone, Sulfoxide, Aminoxide oder Pyridin-N-oxide überführt.

3. Verfahren nach Anspruch 2,
dadurch gekennzeichnet,
daß man die erhaltenen Verbindungen in ihre physiologisch verträglichen Salze überführt.

4. Arzneimittel,
dadurch gekennzeichnet,
daß es als Wirkstoff mindestens eine Verbindung nach einem oder mehreren der vorangegangenen Ansprüche zusammen mit einem üblichen pharmazeutischen Träger und/oder Verdünnungsmittel enthält.

5. Verfahren zur Herstellung eines Arzneimittels,
dadurch gekennzeichnet,
daß eine Verbindung nach einem oder mehreren der vorangegangenen Ansprüche mit gebräuchlichen pharmazeutischen Trägerstoffen beziehungsweise Verdünnungsmitteln zu pharmazeutischen Zubereitungen verarbeitet wird.

6. Verwendung von Verbindungen nach einem oder mehreren der vorangegangenen Ansprüche zur Herstellung eines Arzneimittels.

7. Verwendung von Verbindungen nach einem oder mehreren der vorangegangenen Ansprüche zur Bekämpfung von Schmerzzuständen.